# EUROPÄISCHE PATENTSCHRIFT

(11) **EP 0 891 748 B1**
(45) Veröffentlichungstag und Bekanntmachung des Hinweises auf die Patenterteilung: **21.08.2002**
(21) Anmeldenummer: 98112827.5
(22) Anmeldetag: 10.07.1998
(51) Int. Cl.: A61C 8/00

(54) **Gingivaformer**
Device for forming gum tissue
Dispositif favorisant la régénération de tissu gingival

(30) Priorität: 17.07.1997 DE 19730596
(43) Veröffentlichungstag der Anmeldung: 20.01.1999
(73) Patentinhaber: FRIADENT GmbH, 68229 Mannheim (DE)
(72) Erfinder: Fouad, Khoury, Prof. Dr., 48161 Münster (DE)
(74) Vertreter: Schmitt, Meinrad, Dipl.-Ing.

(56) Entgegenhaltungen:
- DE-A- 3 300 764
- US-A- 4 856 994
- US-A- 4 872 840
- US-A- 4 988 351

## Beschreibung

Die Erfindung bezieht sich auf einen Gingivaformer gemäß den im Oberbegriff des Patentanspruchs 1 angegebenen Merkmalen.

Aus der US-A-4 856 994 ist ein derartiger Gingivaformer bekannt, welcher mit einem Dentalimplantat verbindbar ist. Nach einer vorgegebenen Einheilphase des Dentalimplantats erfolgt mittels Skalpell, Elektrotom oder Schleimhautstanze eine Wiedereröffnung, damit eine mit dem Dentalimplantat verbundene Verschlußschraube aus dem Implantat entfernt und durch den Gingivaformer ersetzt werden kann. Der vorbekannte Gingivaformer weist eine zylindrische Außenkontur auf, doch sind heute auch Gingivaformer mit anderen Außenkonturen im Einsatz. In einer auf die Wiedereröffnung folgenden Abheilphase ermöglicht der Gingivaformer die funktionsgerechte Ausbildung des Schleimhautsaums. Es soll sichergestellt werden, daß sich die Gingiva nach dem Einsetzen des Kronenaufbaus bakteriendicht an dessen Gingivabund anlegt. Für Dentalimplantate mit unterschiedlichen Durchmessern werden entsprechend abgestimmte Gingivaformer bereitgestellt und auch entsprechend der jeweiligen Gingivahöhe stehen Gingivaformer in entsprechender Höhe zur Verfügung. Es können jedoch Probleme auftreten, wenn während der Abheilphase die Anpassung der Schleimhaut und Ausbildung des Schleimhautsaums entsprechend der Außenkontur des Gingivaformers nicht in der erwünschten Weise erfolgt. So kann durch lokales Abheben von Bereichen der Schleimhaut deren Anpassung an die Gingivaformer nur unvollständig erfolgen. Auch können unzulässig große Spalte zwischen Gingivaformer und Schleimhaut entstehen, so daß nach dem Ausheilen der Gingiva, dem Einsetzen des Übertragungsaufbaus sowie nach Vollendung der Suprastruktur der erwünschte dichte Verschluß nicht dauerhaft gewährleistet werden kann.

Ferner ist aus der DE-A-33 00 764 ein Gingivaformer bekannt, welcher in seiner dem Dentalimplantat abgewandten Stirnfläche eine Gewindebohrung aufweist. In diese Gewindebohrung kann zum Einsetzen oder Abnehmen des Gingivaformers in das oder von dem Dentalimplantat ein Schlüssel eingesetzt werden.

Desweiteren ist aus der US-A-4 872 840 ein Dentalimplantat mit einem Aufbaukörper für einen Kronenaufbau oder eine Suprastruktur bekannt. Sobald eine vorgegebene Einheilphase des Implantats im Kieferknochen abgelaufen ist, wird die Gingiva im Bereich des Implantats geöffnet und der genannte Aufbaukörper mit dem Implantat verbunden. Der Aufbaukörper enthält im oberen Ende Schlitze für Fäden bzw. Befestigungsmittel an der Gingiva, um eine zusätzliche Stabilisierung des Implantats während einer weiteren Heilungsperiode zu erhalten. Nach Ablauf dieser Heilungsperiode werden die Fäden entfernt und auf dem unverändert mit dem Implantat verbundenen Aufbaukörper wird eine Kappe für einen Kronenaufbau oder für eine Suprastruktur angeordnet. Hierbei wird die Gingiva im Bereich eines unteren Randes der genannten Kappe freigelegt, so daß nachfolgend die Gingiva an den unteren Rand der Kappe bzw. den Kronenaufbau in einer weiteren Phase anheilen muß, in welcher eine Gefährdung des Heilungsprozesses, insbesondere durch eindringende Bakterien, gegeben ist.

Der Erfindung liegt daher die Aufgabe zugrunde, die aufgezeigten Nachteile zu vermeiden und den Gingivaformer der eingangs genannten Art dahingehend weiterzubilden, daß die Ausformung des Weichgewebes verbessert wird. Der Gingivaformer soll eine einfache und funktionsgerechte Handhabung ermöglichen. Des weiteren soll der Gingivaformer einen geringen Herstellungs- und Fertigungsaufwand erfordern.

Die Lösung dieser Aufgabe erfolgt gemäß den im Patentanspruch 1 angegebenen Merkmalen.

Der erfindungsgemäße Gingivaformer zeichnet sich durch eine funktionsgerechte Konstruktion und einen einfachen Aufbau aus. Er enthält in seinem Kopfteil wenigstens eine Durchgangsbohrung, an welcher Befestigungsmittel für die Gingiva anbringbar sind. Durch die Durchgangsbohrung können übliche zahnärztliche Materialien hindurchgezogen und mit entsprechend präparierten Schleimhautlappen vernäht werden. In einer bevorzugten Ausführungsform durchdringen die Durchgangsbohrungen den Kopfteil zweckmäßig unter einem vorgegebenen Winkel bezüglich einer Längsachse vorzugsweise derart, daß das eine Ende in der Seitenwand und das andere Ende in der oberen Endfläche liegt. Besitzt der Gingivaformer in der oberen Endfläche eine Eingriffsöffnung für einen Schraubendreher oder dergleichen, so wird die wenigstens eine Durchgangsbohrung in zweckmäßiger Weise entsprechend beabstandet hierzu angeordnet. Alternativ kann die Durchgangsbohrung radial in der Eingriffsöffnung oder einer axialen Durchgangsbohrung enden. In einer weiteren bevorzugten Ausführungsform ist die wenigstens eine Durchgangsbohrung in einer zur Längsachse im wesentlichen orthogonalen Radialebene angeordnet. Hierbei endet die Durchgangsbohrung in bevorzugter Weise in der koaxial zur Längsachse vorgesehenen Eingriffsöffnung oder einer axialen Durchgangsöffnung für eine Okklusalschraube, mittels welcher der Gingivaformer im Dentalimplantat bzw. Implantatkörper befestigbar ist.

Die Durchgangsbohrungen sind im Bereich der äußeren Seitenwand des Gingivaformers angeordnet bzw. beginnen an der äußeren Seitenwand. Des weiteren sind die Durchgangsbohrung zweckmäßig im oberen Endbereich des Kopfteils angeordnet, insbesondere in der oberen Hälfte des Kopfteils, vorzugsweise im oberen Drittel anschließend an die freie End- oder Stirnfläche des Kopfteils.

Ausgestaltungen und besondere Weiterbildungen der Erfindung sind in den Unteransprüchen angegeben.

Die Erfindung wird nachfolgend anhand der in der Zeichnung dargestellten Ausführungsbeispiele näher erläutert, ohne daß insoweit eine Beschränkung der Erfindung erfolgt. Es zeigen:
- Fig. 1: eine seitliche Ansicht eines Gingivaformers,
- Fig. 2: eine Aufsicht in Blickrichtung II gemäß Fig. 1,
- Fig. 3: ein weiteres Ausführungsbeispiel mit einem triangulär ästhetischen Kopfteil,
- Fig. 4: einen axialen Schnitt durch ein weiteres Ausführungsbeispiel mit in einer Radialebene angeordneten Durchgangsbohrung,
- Fig. 5: eine seitliche Ansicht des Ausführungsbeispiels gemäß Fig. 4.

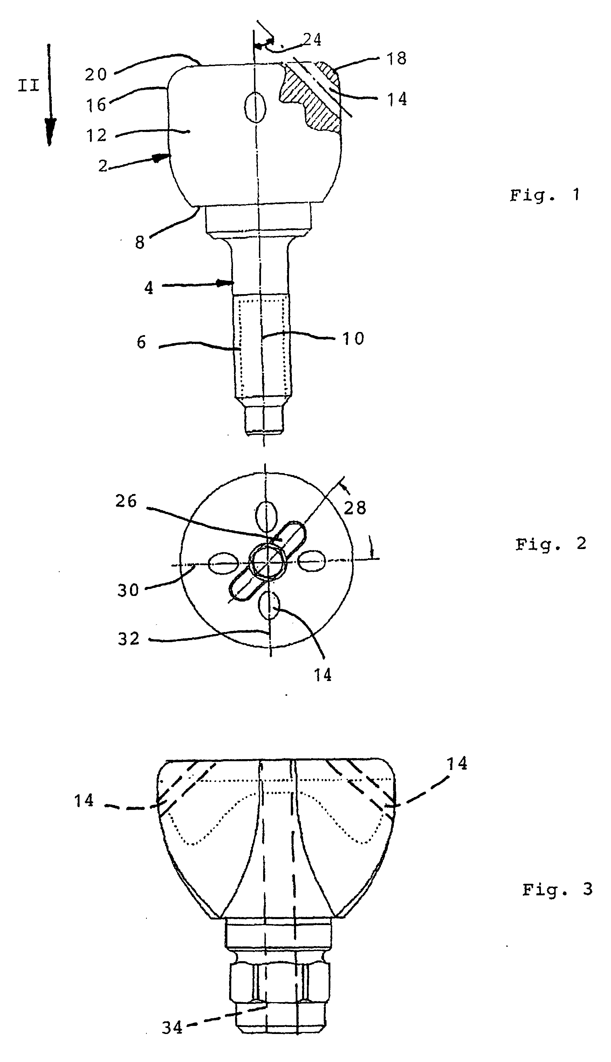

Fig. 1 zeigt in einer seitlichen Ansicht den Gingivaformer mit einem Kopfteil 2 und einem Schaft 4, welcher ein Gewinde 6 aufweist. Der Schaft 4 mit dem Gewinde 6 ist in eine Aufnahmebohrung eines hier nicht weiter dargestellten Implantatkörpers in den Unterkiefer oder Oberkiefer nach der erforderlichen Einheilphase einschraubbar. Der Kopfteil 2 weist an seinem unteren Ende 8 einen Außendurchmesser auf, welcher im wesentlichen dem Außendurchmesser des koronalen Endes des Dentalimplantats bzw. Implantatkörpers entspricht. Ist der Gingivaformer mit dem Implantatkörper verbunden, so ist vom letzteren der Kopfteil 2 frei zugänglich, während der Schaft 4 innerhalb der besagten Aufnahmeöffnung des Implantatkörpers angeordnet ist. Der Kopfteil 2 ist rotationssymmetrisch zur Längsachse 10, wobei die Seitenwand 12 im oberen Bereich zylindrisch und im unteren Bereich karlottenförmig abgerundet ausgebildet ist.

Der Kopfteil enthält mehrere Durchgangsbohrungen 14, weiche eine Fixierung der Schleimhaut mit geeigneten Befestigungsmitteln, wie insbesondere zahnärztliche Nahtmaterialien, ermöglichen. In zweckmäßiger Weise liegt die wenigstens eine Durchgangsbohrung 14 im oberen Endbereich und/oder in der oberen Hälfte, vorzugsweise im oberen Drittel des Kopfteils 2. Wie aus der Schnittdarstellung rechts gemäß Zeichnung gut zu erkennen ist, durchdringt die Durchgangsbohrung die obere Kante 18, wobei das eine Ende in der Seitenwand 12 liegt, während das andere Ende in der oberen Endfläche 20 des Kopfteils 2 liegt. Die Achse 22 der Durchgangsbohrung 14 ist in einem vorgegebenen Winkel 24 zur Längsachse 10 geneigt angeordnet. In zweckmäßiger Weise besitzt der Winkel 24 eine Größe zwischen 30° und 60°, vorzugsweise zwischen 40° und 50°, und liegt insbesondere in der Größenordnung von 45°.

Fig. 2 zeigt eine Aufsicht in Blickrichtung II gemäß Fig. 1, wobei hier die vier Durchgangsbohrung 14 gut zu erkennen sind, welche bezüglich der Längsache gleichmäßig über den Umfang verteilt angeordnet sind. In der oberen Endfläche 20 ist ferner eine Eingriffsöffnung 26 für einen Schraubendreher oder dergleichen zum Einschrauben des Gingivaformers in das Dentalimplantat angeordnet. Die Durchgangsbohrungen 14 enden in der oberen Endfläche 20 in einem vorgegebenen Abstand zur Eingriffsöffnung 26. Bei der hier dargestellten besonderen Ausführungsform sind vier Durchgangsbohrungen 14 bezüglich der zur Zeichenebene orthogonalen Längsachse jeweils um 90° versetzt zueinander angeordnet. Die zumindest teilweise als Schlitz ausgebildete Eingriffsöffnung 26 ist unter einem Winkel 28, welcher im wesentlichen 45° groß ist, bezüglich einer Axialebene 30 angeordnet, in welcher zwei der genannten diametralen Bohrungen 14 liegen. Die beiden anderen diametralen Bohrungen 14 liegen gleichfalls in einer gemeinsamen Axialebene 32.

Fig. 3 zeigt eine weitere Ausführungsform des Gingivaformers, dessen Kopfteil 5 triangulär mit ästhetischer Kontur ausgebildet ist. Dieser Gingivaformer besitzt eine zentrale, axiale Durchgangsbohrung 34, durch welche eine in den hier nicht weiter dargestellten Implantatkörper einschraubbare Halteschraube durchführbar ist. Im Bereich der oberen Kante 18 des Kopfteiles 2 sind die Durchgangsbohrungen 14 zur Fixierung entsprechend präparierter Schleimhautlappen angeordnet.

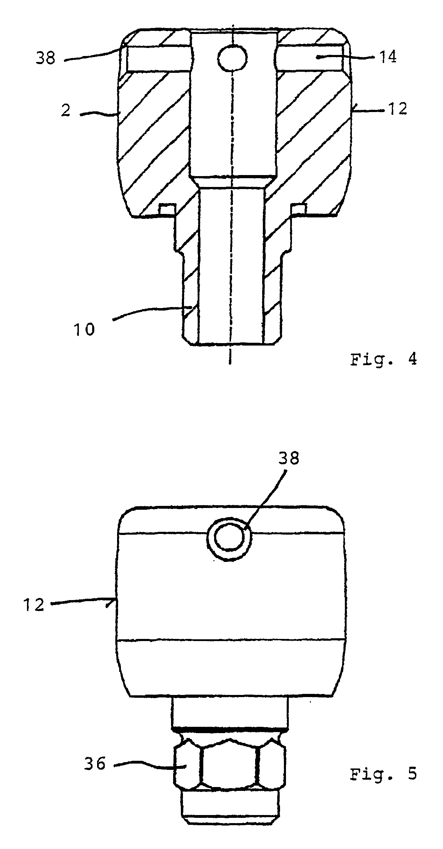

Fig. 4 zeigt eine bevorzugte Ausführungsform mit einer axialen Bohrung 34 zur Festlegung des Gingivaformers mittels einer Halte- oder Okklusalschraube auf dem Implantatkörper. Im Bereich des oberen Endes des Gingivaformers ist wenigstens eine Durchgangsbohrung 14 vorgesehen, welche im wesentlichen in einer Radialebene bezüglich der Längsachse 10 liegt. Bei dem hier gezeigten Ausführungsbeispiel sind insgesamt vier derartige radiale Durchgangsbohrungen 14 vorgesehen, welche jeweils radial innen in der axialen Durchgangsbohrung 34 enden. Die jeweils anderen, radial äußeren Enden der Durchgangsbohrung 14 liegen in der äußeren Seitenwand 12. Dort sind die radialen Durchgangsbohrungen 14 mittels Fasen 38 nach außen erweitert, so daß das Einbringen der Befestigungsmittel für die Gingiva erleichtert wird.

Wie aus Fig. 5 ersichtlich, enthält der in den Implantatkörper eingreifende Schaft 4 seitliche Abflachungen 36 in Form eines Hexagon zwecks Rotationssicherung des Gingivaformers bezüglich des hier nicht dargestellten Implantatkörpers, in dessen korrespondierende Aufnahmeöffnung der Schaft des Gingivaformers eingreift. Selbstverständlich kann der Schaft 4 auch andere Mittel zur Rotationssicherung aufweisen. Desweiteren kann der erfindungsgemäße Gingivaformer auch ohne Schaft ausgebildet sein und in einer dem Implantatkörper zugewandten Stirnfläche eine Ausnehmung zur Rotationssicherung und Ausrichtung zum Implantatkörper enthalten. In diese Ausnehmung greift ein korrespondierender Zapfen des Implantatkörpers zwecks Rotationssicherung und Ausrichtung des Gingivaformers ein.

### Bezugszeichen

- 2: Kopfteil
- 4: Schaft
- 6: Gewinde
- 8: unteres Ende von 2
- 10: Längsachse
- 12: Seitenwand
- 14: Öffnung / Durchgangsbohrung
- 16: oberer Endbereich
- 18: obere Kante
- 20: obere Endfläche
- 22: Achse
- 24: Winkel
- 26: Eingriffsöffnung
- 28: Winkel
- 30, 32: Axialebene
- 34: axiale Bohrung
- 36: Abflachung
- 38: Fase

## Patentansprüche

1. Gingivaformer, welcher mit einem Dentalimplantat verbindbar ist, enthaltend einen dem Bereich der Gingiva anzuordnenden Kopfteil (2),
**dadurch gekennzeichnet, daß** der Kopfteil (2) wenigstens eine Durchgangsbohrung (14) für Befestigungsmittel der Gingiva aufweist.

2. Gingivaformer nach Anspruch 1, **dadurch gekennzeichnet, daß** die Durchgangsbohrung (14) im oberen Endbereich (16) des Kopfteils (2) angeordnet ist, insbesondere in der oberen Hälfte, vorzugsweise im oberen Drittel anschließend an die freie Endfläche (20) des Kopfteils (2).

3. Gingivaformer nach Anspruch 1 oder 2, **dadurch gekennzeichnet, daß** um die Längsachse (10) im wesentlichen gleichmäßig verteilt eine vorgegebene Anzahl von Durchgangsbohrungen (14), insbesondere vier, vorgesehen sind.

4. Gingivaformer nach einem der Ansprüche 1 bis 3, **dadurch gekennzeichnet, daß** die wenigstens eine Durchgangsbohrung (14) in einer Axialebene (30, 32) angeordnet ist.

5. Gingivaformer nach einem der Ansprüche 1 bis 4, **dadurch gekennzeichnet, daß** die Durchgangsbohrung (14) in einem vorgegebenen Winkel (24) bezüglich der Längsachse (10) des Kopfteils (2) angeordnet ist.

6. Gingivaformer nach Anspruch 5, **dadurch gekennzeichnet, daß** der Winkel (24) im Bereich zwischen 30° bis 60°, vorzugsweise zwischen 40° bis 50°, groß ist und im wesentlichen in der Größenordnung von 45° liegt.

7. Gingivaformer nach einem der Ansprüche 1 bis 6, **dadurch gekennzeichnet, daß** die Durchgangsbohrung (14) in einer Radialebene bezüglich der Längsachse (10) angeordnet ist.

8. Gingivaformer nach Anspruch 7, **dadurch gekennzeichnet, daß** die Durchgangsbohrung (14) radial innen in einer axialen Eingriffsöffnung oder axialen Bohrung (34) endet.

9. Gingivaformer nach einem der Ansprüche 1 bis 8, **dadurch gekennzeichnet, daß** die Durchgangsbohrung radial außen in der Seitenwand (12) des Kopfteils (2) endet.

## Claims

1. Device for forming gum tissue which may be combined with a dental implant, containing a head part (2) to be arranged in the region of the gum tissue, **characterised in that** the head part (2) has at least one through-aperture (14) for gum tissue fastening means.

2. Device for forming gum tissue according to claim 1, **characterised in that** the through-aperture (14) is arranged in the upper end region (16) of the head part (2), in particular in the upper half, preferably in the upper third, adjoining the free end face (20) of the head part (2).

3. Device for forming gum tissue according to claim 1 or 2, **characterised in that** a predetermined number of through-apertures (14), in particular four, are provided substantially uniformly distributed about the longitudinal axis (10).

4. Device for forming gum tissue according to any of claims 1 to 3, **characterised in that** the at least one through-aperture (14) is arranged in an axial plane (30, 32).

5. Device for forming gum tissue according to any of claims 1 to 4, **characterised in that** the through-aperture (14) is arranged at a predetermined angle (24) with respect to the longitudinal axis (10) of the head part (2).

6. Device for forming gum tissue according to claim 5, **characterised in that** the angle (24) is in the range between 30° to 60°, preferably between 40° to 50°, and is substantially approximately 45°.

7. Device for forming gum tissue according to any of claims 1 to 6, **characterised in that** the through-aperture (14) is arranged in a radial plane with respect to the longitudinal axis (10).

8. Device for forming gum tissue according to claim 7, **characterised in that** the through-aperture (14) ends radially inwardly in an axial engagement opening or axial aperture (34).

9. Device for forming gum tissue according to any of claims 1 to 8, **characterised in that** the through-aperture ends radially outwardly in the side wall (12) of the head part (2).

## Revendications

1. Dispositif favorisant la régénération du tissu gingival, pouvant être relié à un implant dentaire, comprenant une tête (2) à associer à une zone de la gencive, ***caractérisé en ce que*** la tête (2) présente au moins un perçage traversant (14) pour des moyens de fixation de la gencive.

2. Dispositif favorisant la régénération du tissu gingival selon la Revendication 1, ***caractérisé en ce que*** le perçage traversant (14) est placé dans la zone d'extrémité supérieure (16) de la tête (2), en particulier dans la moitié supérieure, de préférence dans le tiers supérieur à la suite de la surface d'extrémité libre (20) de la tête (2).

3. Dispositif favorisant la régénération du tissu gingival selon la Revendication 1 ou 2, ***caractérisé en ce qu****'un* nombre prédéterminé de perçages traversants (14), en particulier quatre, est prévu pour l'essentiel d'une manière uniformément répartie autour de l'axe longitudinal (10).

4. Dispositif favorisant la régénération du tissu gingival selon l'une quelconque des Revendications 1 à 3, ***caractérisé en ce que*** le perçage traversant (14) au nombre d'au moins un est placé dans un plan axial (30, 32).

5. Dispositif favorisant la régénération du tissu gingival selon l'une quelconque des Revendications 1 à 4, ***caractérisé en ce que*** le perçage traversant (14) est placé suivant un angle prédéterminé (24) par rapport à l'axe longitudinal (10) de la tête (2).

6. Dispositif favorisant la régénération du tissu gingival selon la Revendication 5, ***caractérisé en ce que*** l'angle (24) est compris entre 30° et 60°, de préférence entre 40° et 50°, et est pour l'essentiel de l'ordre de 45°.

7. Dispositif favorisant la régénération du tissu gingival selon l'une quelconque des Revendications 1 à 6, ***caractérisé en ce que*** le perçage traversant (14) est placé dans un plan radial par rapport à l'axe longitudinal (10).

8. Dispositif favorisant la régénération du tissu gingival selon la Revendication 7, ***caractérisé en ce que*** le perçage traversant (14) se termine radialement à l'intérieur dans une ouverture de prise axiale ou un perçage axial (34).

9. Dispositif favorisant la régénération du tissu gingival selon l'une quelconque des Revendications 1 à 8, ***caractérisé en ce que*** le perçage traversant se termine radialement à l'extérieur dans la paroi latérale (12) de la tête (2).
